# EUROPEAN PATENT APPLICATION

(11) **EP 3 858 971 A1**
(43) Date of publication of application: **04.08.2021**
(21) Application number: 19868007.6
(22) Date of filing: 27.09.2019
(51) Int. Cl.: C12M 1/24, C12M 1/12, C12M 1/00, G01N 33/50

(54) **BIOCHIP, MICRO LIQUID SAMPLE LOADING STRUCTURE THEREOF, AND MICRO QUANTITATIVE SAMPLING METHOD**

(30) Priority: 28.09.2018 CN 201811135415; 28.09.2018 CN 201811136983
(71) Applicant: Hunan Legend Ai Chip Biotechnology Co., Ltd., Changsha, Hunan 410205 (CN)
(72) Inventor: WU, Pengcheng, Changsha, Hunan 410205 (CN); ZHOU, Xiaoxiang, Changsha, Hunan 410205 (CN); XIANG, Sumin, Changsha, Hunan 410205 (CN); YANG, Hengwei, Changsha, Hunan 410205 (CN)
(74) Representative: Grünecker Patent- und Rechtsanwälte PartG mbB
(86) International application number: PCT/CN2019/108336
(87) International publication number: WO 2020/063795

(57) **Abstract**

The present invention discloses a biochip, a micro liquid sample loading structure thereof, and a micro quantitative sampling method. The micro liquid sample loading structure comprises a quantitative tube and a hemofiltration structure, wherein a side wall of the quantitative tube is provided with a sample injection port; the hemofiltration structure comprises a housing, a filtration membrane is arranged in the housing, a top of the housing is provided with a first cavity with an upward opening, a second cavity is formed in the housing, the first cavity is communicated with the second cavity by the filtration membrane, and the filtration membrane is perpendicular to a horizontal plane; and the second cavity is communicated with the sample injection port. The micro liquid sample loading structure can be easily and directly integrated in the biochip, and can prevent air from being brought into a reactor; the filtration membrane is not easily blocked by red blood cells during the preparation of a plasma sample, the hemofiltration time is short, the efficiency is high, and the risk of crushing red blood cells is low; quantification and sampling are completed in the same process; sample droplets adhered to and hung at the sample outlet at the end of the quantitative tube are blown into the reactor in a non-contact manner, so that the sample will not be contaminated, and the quantitative precision of the sample is high; and the structure is simple, the cost is low, and the stability is good.

## Description

### FIELD OF THE INVENTION

The present invention belongs to the field of biochip detection, and more particularly to a biochip, a micro liquid sample loading structure thereof, and a micro quantitative sampling method.

### BACKGROUND OF THE INVENTION

Biochip technology has been rapidly developed in the recent 20 years. Especially with the rapid development of microfluidic technology, new material technology and artificial intelligence technology, the biochip technology has gradually moved towards industrialization. However, micro liquid sample loading and micro quantitative sampling in the biochip are now a universal problem.

The existing micro liquid sample loading structure includes a quantitative tube. During use, air in the quantitative tube is first removed, and then micro liquid is quantitatively sucked into the quantitative tube from a container based on the principle of negative pressure to complete a sample loading process. When sampling, the quantitative tube is displaced (moved or rotated) to a designated position, and then the liquid is pushed out of the quantitative tube by means of air to achieve the purpose of sampling.

The existing micro liquid sample loading structure needs to be displaced during the sampling process, so such structure is difficult or unable to integrate in a small biochip.

In this way, air is easily sucked into the quantitative tube, inevitably mixed with the liquid and pushed into a reactor of the biochip to affect the detection result of the sample by the biochip.

Moreover, in the prior art, plasma samples used in biochip detection are all prepared using a hemofiltration structure in the form of vertical filtration. The hemofiltration structure includes a housing, a filtration membrane placed horizontally is arranged in the housing, a top of the housing is provided with a first cavity with an upward opening, a second cavity right below the first cavity is formed in the housing, and the first cavity is communicated with the second cavity by the filtration membrane. When blood is filtered, the whole blood is first added to the first cavity, then the first cavity is sealed and pressurized, small molecular plasma will pass through the filtration membrane below the first cavity and enter the second cavity, and large molecular red blood cells will be retained in the first cavity, thereby achieving the purpose of filtering blood.

In the vertical hemofiltration structure, since red blood cells are retained in the first cavity and are deposited on the filtration membrane at the bottom of the first cavity, the concentration of red blood cells in the first cavity is increasingly high as hemofiltration goes on. As a result, the filtration membrane is easily blocked, the blood cannot be filtered thoroughly, the time for filtering a unit amount of sample becomes longer, the efficiency is reduced, and even red blood cells are crushed to cause sample failure.

The existing way can realize the quantification of whole blood. However, the whole blood hematocrits of different people are different, and the quantitative detection of whole blood will cause inaccurate filtered plasma due to the different hematocrits, which directly results in the inaccurate detection results.

### SUMMARY OF THE INVENTION

Aiming at the shortcomings of the prior art, the purpose of the present invention is to provide a biochip, a micro liquid sample loading structure thereof, and a micro quantitative sampling method.

The existing micro liquid sample loading structure needs to be displaced during the sampling process, so such structure is difficult or unable to be integrated in a small biochip. In addition, air is easily sucked into the quantitative tube, inevitably mixed with the liquid and pushed into a reactor of the biochip to affect the detection result of the sample by the biochip. In order to solve this technical problem, the technical solution adopted by the present invention is:
A micro liquid sample loading structure, including a quantitative tube and a hemofiltration structure, its structural features are that a quantitative plunger for sealing an inner cavity of the quantitative tube is arranged at a top of the quantitative tube, a sample injection port communicated with the inner cavity of the quantitative tube is formed at an upper part of a side wall of the quantitative tube, and a sample outlet is formed at a bottom of the quantitative tube; and a plasma outlet of the hemofiltration structure is communicated with the sample injection port.

With the above structure, after the hemofiltration structure filters whole blood to obtain a plasma sample, the plasma sample is injected into the inner cavity of the quantitative tube from the plasma outlet of the hemofiltration structure through the sample injection port. Because the quantitative tube is sealed at the upper end and opened at the lower end, the sample automatically enters the inner cavity of the quantitative tube. During sample injection, air in the quantitative tube is continuously pushed out from the sample outlet by the sample, and the amount of the sample injected into the inner cavity of the quantitative tube is controlled, and an air-free sample liquid column is finally formed in the quantitative tube, which provides a possibility for accurate quantitative sampling.

Finally, the quantitative plunger is pressed down to push the sample out of the quantitative tube on demand. Because the present invention does not need to discharge air to form negative pressure for sucking a sample, the micro liquid sample loading structure can be easily and directly integrated in a small biochip. Because the air-free liquid column can be formed in the quantitative tube, when air entrained in the sample enters the reactor of the biochip during sample adding, any adverse effect will not be produced on the detection result of the sample.

The micro quantitative of the present invention indicates that the volume of sample pushed out from the sample outlet of the quantitative tube every time is 3 µl to 100 µl.

As a preferred manner, the bottom of the quantitative tube is conical, and the sample outlet is arranged at a lowest point of the bottom of the quantitative tube. This form facilitates automatic entry of the sample into the inner cavity of the quantitative tube and discharge of air from the bottom sample outlet, which is more advantageous to form the air-free sample liquid column.

As a preferred manner, in the initial injection state, a bottom surface of the quantitative plunger is coplanar with a highest point of the sample injection port; or the bottom surface of the quantitative plunger is located between the highest point and a lowest point of the sample injection port.

When the bottom surface of the quantitative plunger is located between the highest point and the lowest point of the sample injection port, it is more advantageous to form the air-free sample liquid column.

As a preferred manner, the quantitative tube and the hemofiltration structure are integrated.

In this way, different hematocrits of different people do not need be considered, only the whole blood is added, and a micro quantitative plasma sample can be obtained by the single structure to realize accurate detection of the sample.

As a preferred manner, height of the sample injection port is 1 mm to 2.5 mm, and the diameters of a middle section and an upper section of the quantitative tube are 0.5 mm to 3.5 mm. The diameter of the quantitative tube is designed according to the viscosity of fluid (plasma), which facilitates the discharge of air during sample injection.

Plasma samples used in existing biochip detection are all prepared using a hemofiltration structure in the form of vertical filtration. Since red blood cells are retained in the first cavity and deposited on the filtration membrane at the bottom of the first cavity, the concentration of red blood cells in the first cavity is increasingly high as hemofiltration goes on. As a result, the filtration membrane is easily blocked, the blood cannot be filtered thoroughly, the time for filtering a unit amount of sample becomes longer, the efficiency is reduced, and even red blood cells are possible to be crushed to cause sample failure. In order to solve this technical problem, the technical solution adopted by the present invention is:
Further, the hemofiltration structure includes a housing, a filtration membrane is arranged in the housing, a top of the housing is provided with a first cavity with an upward opening, a second cavity is formed in the housing, the first cavity is communicated with the second cavity by the filtration membrane, and the filtration membrane is perpendicular to a horizontal plane; and the second cavity is communicated with the sample injection port.

With the above structure, since the filtration membrane is perpendicular to the horizontal plane, compared to horizontal placement of the filtration membrane in the prior art, the filtration membrane is vertically arranged on a side of the first cavity in the present invention. During hemofiltration, even if red blood cells are retained in the first cavity and deposited at the bottom of the first cavity, the red blood cells are unlikely to block the filtration membrane vertically arranged on the side of the first cavity, so the plasma passes through the filtration membrane smoothly, the hemofiltration is thorough, the hemofiltration time is short, the efficiency is high, and the risk of crushing red blood cells is low.

Further, a hematocrit cavity is further formed in the housing, the hematocrit cavity is communicated with the bottom of the first cavity, the volume of the hematocrit cavity is smaller than the volume of the first cavity, the hematocrit cavity is communicated with the second cavity by the filtration membrane, and the volume of the hematocrit cavity is greater than or equal to the total volume of red blood cells in whole flood to be filtered.

With the above structure, the bottom of the first cavity is communicated with the hematocrit cavity, the whole blood to be filtered in the first cavity is continuously delivered into the hematocrit cavity, then the plasma passes through the filtration membrane and enters the second cavity, and the red blood cells are retained in the hematocrit cavity to achieve the purpose of hemofiltration. Since the volume of the hematocrit cavity is greater than or equal to the total volume of red blood cells in the whole flood to be filtered, the red blood cells are deposited at the bottom of the hematocrit cavity during hemofiltration, the upper space of the hematocrit cavity is reserved to receive newly injected whole blood, the filtration membrane is more unlikely to be blocked by red blood cells, and the hemofiltration is smoother.

Further, the micro liquid sample loading structure further includes a hemofiltration plunger that can seal the top opening of the first cavity.

During hemofiltration, a driving force is applied to the hemofiltration plunger by means of a driving mechanism, so that the hemofiltration plunger seals and pressurizes the first cavity, causing the whole blood to flow toward the filtration membrane to achieve the purpose of hemofiltration.

As a preferred manner, a first highest point, on the side of the filtration membrane where the filtration membrane is connected to the hematocrit cavity, is on the same horizontal plane as a second highest point on the opposite side of the filtration membrane; or the first highest point, on the side of the filtration membrane where the filtration membrane is connected to the hematocrit cavity, is higher than the second highest point on the opposite side of the filtration membrane.

When the first highest point, on the side of the filtration membrane where the filtration membrane is connected to the hematocrit cavity, is higher than the second highest point on the opposite side of the filtration membrane, the filtration membrane is more unlikely to be blocked.

Based on the same inventive concept, the present invention further provides a biochip, including a reactor and the micro liquid sample loading structure, wherein the sample outlet is located above the reactor.

In the existing biochip, quantifying and sampling are two separate processes, and the sample needs to be displaced in the sampling process after the quantifying process, so the micro liquid sample loading structure is difficult to be integrated in the small biochip.

Meanwhile, by means of pushing the liquid out with air, the air is inevitably mixed with the liquid and pushed into the reactor of the biochip to affect the detection result of the sample by the biochip. In order to solve this technical problem, the technical solution adopted by the present invention is:
Further, the biochip further includes a chip substrate, and the reactor and the hemofiltration structure are arranged in the chip substrate; the quantitative tube is arranged in the chip substrate; the sample outlet is located above the reactor; the biochip further includes a controller and a driving mechanism that can drive the quantitative plunger to move along the axis of the quantitative tube, the controller is electrically connected to the driving mechanism, and the driving mechanism is connected to the quantitative plunger in a driving manner.

With the above structure, sample liquid is injected to the inner cavity of the quantitative tube from the sample injection port. Because the quantitative tube is sealed at the upper end and opened at the lower end, the sample automatically enters the inner cavity of the quantitative tube. The diameter of the quantitative tube is designed according to the viscosity of fluid (plasma), which facilitates the discharge of air during sample injection.

Air in the quantitative tube is continuously pushed out from the sample outlet by the sample, and the amount of the sample injected into the inner cavity of the quantitative tube is controlled, and an air-free sample liquid column is finally formed in the quantitative tube, which provides a possibility for accurate quantitative sampling.

Subsequently, the quantitative plunger is driven by the driving mechanism to move down, and the sample in the quantitative tube can be quantitatively pushed out on demand by controlling the stroke of the quantitative plunger. Because the present invention does not need to discharge air to form negative pressure for sucking a sample, the micro liquid sample loading structure can be easily and directly integrated in a small biochip; the air-free liquid column can be formed in the quantitative tube, which avoids adding the sample carrying air to the reactor of the biochip and will not produce any adverse effect on the detection result of the sample; and quantifying and sampling can be completed in the same process by controlling the stroke of the quantitative plunger, so the operation is simple and convenient.

Further, the biochip further includes a camera unit, the quantitative tube is made of a transparent material and located within the field of view of the camera unit, and the camera unit is electrically connected to the controller.

The camera unit can capture sample images in the quantitative tube, and the controller can calculate the injection amount and ejection amount of the sample through the sample images captured, thereby achieving accurate control.

Further, the biochip further includes a blowing unit, the housing is provided with a mounting through hole, the lower section of the quantitative tube extends into the mounting through hole, a side wall of the housing is provided with a blowing through hole that communicates an air outlet of the blowing unit with the lower section of the quantitative tube, and an air passage that communicates the blowing through hole with the outer wall of the sample outlet is formed between the lower section of the quantitative tube and the mounting through hole.

After the quantitative tube quantitatively pushes the sample liquid out each time, quantitative micro sample droplets are adhered to and hung at the sample outlet. At this time, the blowing unit blows air toward the sample outlet via the blowing through hole and the air passage in sequence, to blow away the quantitative micro sample droplets adhered to the sample outlet, which avoids contaminating the sample and ensures the quantitative precision of the sample.

Based on the same inventive concept, the present invention further provides a micro quantitative sampling method for the biochip, including the following steps:
Step A. Injecting a plasma sample into the inner cavity of the quantitative tube from the sample injection port;
Step B. The plasma sample reaching the bottom of the quantitative tube;
Step C. The quantitative plunger moving down along the axis of the quantitative tube to push a certain amount of plasma sample out from the quantitative tube; and
Step D. The plasma sample entering the reactor.

Further, in step B, when the plasma sample reaches a first scale line on the quantitative tube, the injection of the plasma sample into the quantitative tube is stopped, and meanwhile, the quantitative plunger moves down along the axis of the quantitative tube and pushes the plasma sample down; and when the plasma sample in the quantitative tube reaches a second scale line below the first scale line, the plasma sample reaches the bottom of the quantitative tube.

Further, the controller calculates the stroke of the quantitative plunger to push a certain amount of plasma sample out of the quantitative tube, and the controller drives the quantitative plunger by the driving mechanism according to the calculated stroke.

As a preferred manner, the plasma sample is obtained from whole blood by means of the hemofiltration structure.

Compared with the prior art, the present invention does not need to discharge air to form negative pressure for sucking a sample, so the micro liquid sample loading structure can be easily and directly integrated in a small biochip; the air-free liquid column can be formed in the quantitative tube during sample injection, which avoids entraining air into the reactor of the biochip and will not produce any adverse effect on the detection result of the sample; meanwhile, red blood cells are unlikely to block the filtration membrane when a plasma sample is prepared, so the hemofiltration is thorough, the hemofiltration time is short, the efficiency is high, the risk of crushing red blood cells is low, the sample is not easily contaminated, and the present invention is especially suitable for the preparation of biochip plasma test samples, greatly reduces test cost and improves test efficiency; quantifying and sampling are completed in the same process; quantitative micro sample droplets adhered to and hung at the sample outlet at the end of the quantitative tube are blown into the reactor of the biochip in a non-contact manner, so that the sample will not be contaminated, and the quantitative precision of the sample is high; and the structure is simple, the cost is low, and the stability is good.

### BRIEF DESCRIPTION OF THE DRAWINGS

Fig. 1 is a schematic structural diagram of an embodiment of a micro liquid sample loading structure.
Fig. 2 is a schematic diagram showing that a filtration membrane is inserted into a housing insertion port.
Fig. 3 is a schematic structural diagram of an embodiment of a biochip.
Fig. 4 is a side cross-sectional view of Fig. 3.
Fig. 5 is a circuit module diagram of the present invention.
Fig. 6 is a flowchart of a micro quantitative sampling method.
Fig. 7 is a sequence diagram of software operations of the micro quantitative sampling method.

In the figures: 1 - housing, 101 - first cavity, 102 - second cavity, 103 - hematocrit cavity, 104 - insertion port, 105 - mounting through hole, 106 - blowing through hole, 2 - filtration membrane, 3 - hemofiltration plunger, 4 - quantitative tube, 401 - sample injection port, 402 - sample outlet, 5 - quantitative plunger, 6 - blowing unit, 7 - air passage, 8 - camera unit, 9 - controller, 10 - reactor, 11 - driving mechanism, 12 - chip substrate, 13 - first scale line, 14 - second scale line, 15 - feed port.

### DETAILED DESCRIPTION OF EMBODIMENTS

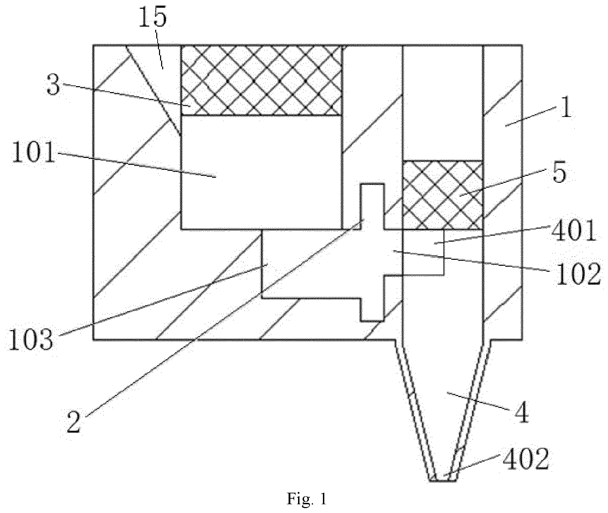

As shown in Fig. 1, a micro liquid sample loading structure includes a quantitative tube 4 and a hemofiltration structure, the quantitative tube 4 and the hemofiltration structure are integrated, and a micro quantitative plasma sample can be obtained from whole blood by means of the hemofiltration structure. A quantitative plunger 5 for sealing an inner cavity of the quantitative tube 4 is arranged at a top of the quantitative tube 4, a sample injection port 401 communicated with the inner cavity of the quantitative tube 4 is formed at an upper part of a side wall of the quantitative tube 4, and a sample outlet 402 is formed at a bottom of the quantitative tube 4; and a plasma outlet of the hemofiltration structure is communicated with the sample injection port 401. The quantitative plunger 5 may be made of different materials and have different thicknesses as required.

After the hemofiltration structure filters whole blood to obtain a plasma sample, the plasma sample is injected into the inner cavity of the quantitative tube 4 from the plasma outlet of the hemofiltration structure through the sample injection port 401. Because the quantitative tube 4 is sealed at the upper end and opened at the lower end, the sample automatically enters the inner cavity of the quantitative tube 4. During sample injection, air in the quantitative tube 4 is continuously pushed out from the sample outlet 402 by the sample, and the amount of the sample injected into the inner cavity of the quantitative tube 4 is controlled, and an air-free sample liquid column is finally formed in the quantitative tube 4, which provides a possibility for accurate quantitative sampling. Subsequently, the quantitative plunger 5 is driven by a driving mechanism 11 to move down, and the sample in the quantitative tube 4 can be quantitatively pushed out on demand by controlling the stroke of the quantitative plunger 5. Because the present invention does not need to discharge air to form negative pressure for sucking a sample, the micro liquid sample loading structure can be easily and directly integrated in a small biochip; the air-free liquid column can be formed in the quantitative tube 4, which avoids adding the sample carrying air to a reactor 10 of the biochip and will not produce any adverse effect on the detection result of the sample; and quantifying and sampling can be completed in a same process by controlling the stroke of the quantitative plunger 5, so the operation is simple and convenient.

The bottom of the quantitative tube 4 is conical, and the sample outlet 402 is arranged at a lowest point of the bottom of the quantitative tube 4. This form facilitates automatic entry of the sample into the inner cavity of the quantitative tube 4 and discharge of air from the bottom sample outlet 402, which is more advantageous to form the air-free sample liquid column.

In the initial injection state, a bottom surface of the quantitative plunger 5 is coplanar with a highest point of the sample injection port 401; or the bottom surface of the quantitative plunger 5 is located between the highest point and the lowest point of the sample injection port 401. When the bottom surface of the quantitative plunger 5 is located between the highest point and the lowest point of the sample injection port 401, it is more advantageous to form the air-free sample liquid column.

Height of the sample injection port 401 is 1 mm to 2.5 mm, and the diameters of a middle section and an upper section of the quantitative tube 4 are 0.5 mm to 3.5 mm.

The diameter of the quantitative tube is designed according to the viscosity of fluid (plasma), which facilitates the discharge of air during sample injection. In addition to the structure described in this embodiment, the quantitative tube 5 may also employ other structures, such as a cuboid structure.

The quantitative tube 4 is made of a hydrophobic material, or the inner wall of the quantitative tube 4 has a hydrophobic coating.

The inner surface of the quantitative tube 4 is a mirror surface.

The hemofiltration structure includes a housing 1, a filtration membrane 2 is arranged in the housing 1, a top of the housing 1 is provided with a first cavity 101 with an upward opening, a second cavity 102 is formed in the housing 1, the first cavity 101 is communicated with the second cavity 102 by the filtration membrane 2, and the filtration membrane 2 is perpendicular to a horizontal plane; and the second cavity 102 is communicated with the sample injection port 401. A hematocrit cavity 103 is further formed in the housing 1, the hematocrit cavity 103 is communicated with the bottom of the first cavity 101, the volume of the hematocrit cavity 103 is smaller than the volume of the first cavity 101, the hematocrit cavity 103 is communicated with the second cavity 102 by the filtration membrane 2, and the volume of the hematocrit cavity 103 is greater than or equal to the total volume of red blood cells in whole flood to be filtered.

Preferably, the filtration membrane 2 is perpendicular to the horizontal plane.

By the amount of plasma need to be obtained by filtration, the volume of the whole blood to be filtered can be calculated, and then the total volume of red blood cells in the whole blood can be obtained. For example, if 20 microliters of plasma is required, because human whole blood contains only 40% to 50% of plasma, at least 50 microliters of whole blood is required. The 50 microliters of whole blood contains 25 to 30 microliters of red blood cells, so the volume of the hematocrit cavity 103 is 25 to 30 microliters or slightly more.

The hematocrit cavity 103 may be in a regular shape such as a square or a cuboid, or in an irregular shape.

The filtration membrane 2 is perpendicular to the horizontal plane, compared to horizontal placement of the filtration membrane 2 in the prior art, the filtration membrane 2 is vertically arranged on a side of the hematocrit cavity 103 in the present invention. The bottom of the first cavity 101 is communicated with the hematocrit cavity 103, the whole blood to be filtered in the first cavity 101 is continuously delivered into the hematocrit cavity 103, then the plasma passes through the filtration membrane 2 and enters the second cavity 102, and the red blood cells are retained in the hematocrit cavity 103 to achieve the purpose of hemofiltration.

Since the volume of the hematocrit cavity 103 is greater than or equal to the total volume of red blood cells in the whole flood to be filtered, the red blood cells are deposited at the bottom of the hematocrit cavity 103 during hemofiltration, and the upper space of the hematocrit cavity 103 is reserved to receive newly injected whole blood. Because the filtration membrane 2 is vertically arranged on the side of the hematocrit cavity 103, the filtration membrane 2 is not easily blocked by red blood cells, the hemofiltration is smoother, the hemofiltration is thorough, the hemofiltration time is short, the efficiency is high, and the risk of crushing red blood cells is low.

The hemofiltration structure further includes a hemofiltration plunger 3 that can seal the top opening of the first cavity 101. The driving mechanism 11 is connected to the hemofiltration plunger 3 in a driving manner.

During hemofiltration, a driving force is applied to the hemofiltration plunger 3 by means of the driving mechanism 11, so that the hemofiltration plunger 3 seals and pressurizes the first cavity 101, causing the whole blood to flow toward the filtration membrane 2 to achieve the purpose of hemofiltration.

In this embodiment, a first highest point, on the side of the filtration membrane 2 where the filtration membrane 2 is connected to the hematocrit cavity 103, is on the same horizontal plane as a second highest point on the opposite side of the filtration membrane 2 (the first highest point, on the side of the filtration membrane 2 where the filtration membrane 2 is connected to the hematocrit cavity 103, can also be higher than the second highest point on the opposite side of the filtration membrane 2, which is not shown in the drawings, but does not affect the understanding and implementation of the present invention by those skilled in the art. When the first highest point, on the side of the filtration membrane 2 where the filtration membrane 2 is connected to the hematocrit cavity 103, is higher than the second highest point on the opposite side of the filtration membrane 2, the filtration membrane 2 is more unlikely to be blocked).

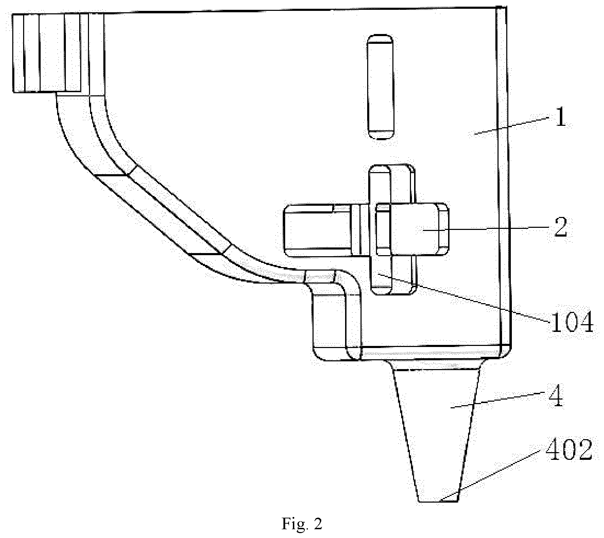

The housing 1 is provided with an insertion port 104 for inserting the filtration membrane 2. In Fig. 2, the insertion port 104 is formed on a side wall of the housing 1, and the insertion port 104 can also be formed at the top or bottom of the housing 1. The edge of the filtration membrane 2 is covered with flexible glue. When mounted, the filtration membrane 2 is in interference fit with a side wall of the insertion port 104 to achieve a sealing effect and prevent sample leakage from the side.

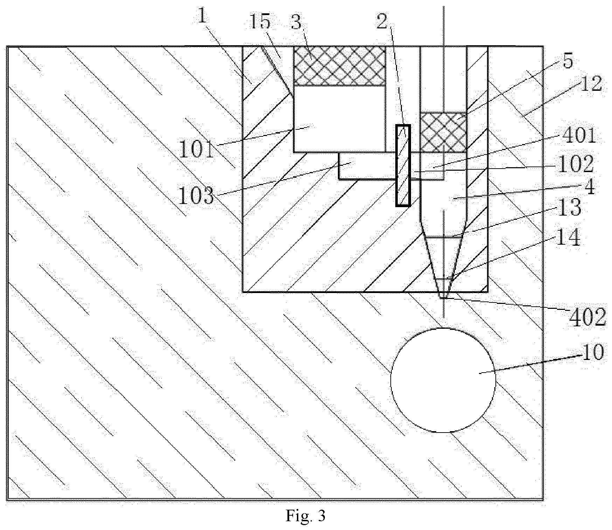

As shown in Figs. 3 to 5, the present invention further provides a biochip, including a reactor 10 and the micro liquid sample loading structure, wherein the sample outlet 402 is located above the reactor 10.

The biochip further includes a chip substrate 12, and the reactor 10 and the hemofiltration structure are arranged in the chip substrate 12; the quantitative tube 4 is arranged in the chip substrate 12; the sample outlet 402 is located above the reactor 10; the biochip further includes a controller 9 and a driving mechanism 11 that can drive the quantitative plunger 5 to move along the axis of the quantitative tube 4, the controller 9 is electrically connected to the driving mechanism 11, and the driving mechanism 11 is connected to the quantitative plunger 5 in a driving manner. The quantitative plunger 5 may be made of different materials and have different thicknesses as required.

The micro liquid sample loading structure can be connected with the reactor 10 into a whole, or the two are independent components that are not connected to each other.

The outer side wall of the quantitative tube 4 is provided with a first scale line 13 and a second scale line 14, and the first scale line 13 is located above the second scale line 14.

The biochip further includes a camera unit 8, the quantitative tube 4 is made of a transparent material and located within the field of view of the camera unit 8, and the camera unit 8 is electrically connected to the controller 9. The camera unit 8 can capture sample images in the quantitative tube 4, and the controller 9 can calculate the injection amount and ejection amount of the sample in the quantitative tube 4 through the sample images received, thereby achieving accurate control.

The biochip further includes a blowing unit 6, the housing 1 is provided with a mounting through hole 105, the lower section of the quantitative tube 4 extends into the mounting through hole 105, a side wall of the housing 1 is provided with a blowing through hole 106 that communicates an air outlet of the blowing unit 6 with the lower section of the quantitative tube 4, and an air passage 7 that communicates the blowing through hole 106 with the outer wall of the sample outlet 402 is formed between the lower section of the quantitative tube 4 and the mounting through hole 105.

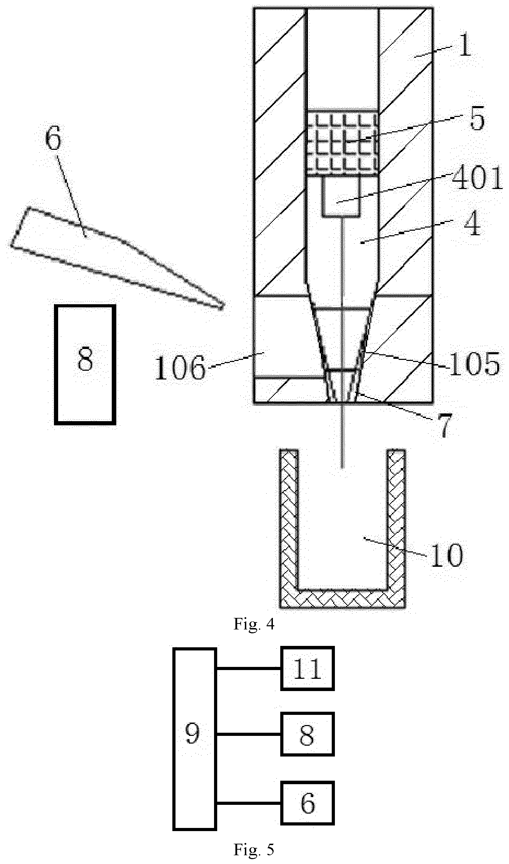

In Fig. 4, the air outlet of the blowing unit 6 is arranged on a side of the blowing through hole 106. In practice, the air outlet of the blowing unit 6 can be set according to the actual situation, that is, the distance between the air outlet of the blowing unit 6 and the housing 1 can be adjusted according to the actual situation, and whether the air outlet of the blowing unit 6 needs to extend into the housing 1 can be set according to the actual situation. In addition, the blowing direction at the air outlet of the blowing unit 6 can also set as required. The blowing of air from the air outlet can be along a lateral direction, a vertical direction or an oblique direction, and air may be blown laterally, vertically, or obliquely.

After the quantitative tube 4 quantitatively pushes the sample liquid out each time, quantitative micro sample droplets are adhered to and hung at the sample outlet 402. At this time, the blowing unit 6 blows air toward the sample outlet 402 via the blowing through hole 106 and the air passage 7 in sequence, thus blow away the quantitative micro sample droplets adhered to the sample outlet 402, which avoids contaminating the sample and ensures the quantitative precision of the sample.

After the droplets are blown away, a small part remains at the end of the quantitative tube 4 (air cannot blow away all the liquid). However, this part of residue is stable each time, and is contained in the sample when the quantitative tube 4 quantitatively discharges the sample, so the quantitative precision will not be affected.

The mounting through hole 105 is a tapered hole with diameters reduced from top to bottom.

The air passage 7 formed in the above structure can change the flow direction of air and effectively gather the air, so that the air is concentrated and blown to upper parts of the droplets adhered to the sample outlet 402, and the droplets can vertically fall to a designated area below the sample outlet 402 and will not be scattered, which further ensures the quantitative precision of the sample.

The middle section of the outer side wall of the quantitative tube 4 fits the housing 1.

Therefore, air can be prevented from flowing up, and the position of the quantitative tube 4 within the housing 1 can also be ensured.

The blowing unit 6 includes an air pump and an air tube connected to an air outlet of the air pump, a control end of the air pump is electrically connected to an output end of the controller 9, and an air outlet of the air tube is opposite to the blowing through hole 106.

A non-contact auxiliary blowing structure further includes the camera unit 8 and the controller 9, the sample outlet 402 is located within the field of view of the camera unit 8, and both the camera unit 8 and the blowing unit 6 are electrically connected to the controller 9.

After the quantitative tube 4 quantitatively pushes the sample liquid out each time, quantitative micro sample droplets are adhered to and hung at the sample outlet 402. At this time, the blowing unit 6 blows air toward the sample outlet 402 via the blowing through hole 106 and the air passage 7 in sequence, thus blow away the quantitative micro sample droplets adhered to the sample outlet 402, which avoids contaminating the sample and ensures the quantitative precision of the sample.

The camera unit 8 can capture droplet images at the sample outlet 402, the camera unit 8 transmits the acquired data to the controller 9, the controller 9 calculates the volume of droplets according to an image processing method, and the controller 9 controls the output amount of the blowing unit 6 according to the volume of droplets, which can ensure that sample droplets of different sizes can be precisely and vertically blown off.

If the volume of liquid droplets is large, the blowing unit 6 blows a lot of air with high pressure for a long time; and if the volume of liquid droplets is small, the blowing unit 6 blows a little air with low pressure for a short time.

The driving mechanism 11 is a mobile motor. The connection relationships of the driving mechanism 11, the hemofiltration plunger 3, and the quantitative plunger 5 are not shown in the drawings, but do not affect the understanding and implementation of the present invention by those skilled in the art.

During hemofiltration, a driving force is applied to the hemofiltration plunger 3 by means of the driving mechanism 11, so that the hemofiltration plunger 3 seals and pressurizes the first cavity 101, causing the whole blood to flow toward the filtration membrane 2 to achieve the purpose of hemofiltration. During operation, the hemofiltration plunger 3 is unable to press the hematocrit cavity 103. Excess sample plasma is filtered to the second cavity 102 from the filtration membrane 2, and the volume of the whole blood is reduced, so the hematocrit cavity 103 is in a pressure-free state, and there is no risk of crushing red blood cells.

A micro quantitative sampling method for the biochip includes the following steps:
Step A. A plasma sample is injected into the inner cavity of the quantitative tube 4 from the sample injection port 401;
Step B. The plasma sample reaches the bottom of the quantitative tube 4 (the bottom of the quantitative tube 4 is provided with a reference position, which is used to provide a position reference for the stroke of the quantitative plunger 5);
Step C. The quantitative plunger 5 moves down along the axis of the quantitative tube 4 to push a certain amount of plasma sample out from the quantitative tube 4;
Step D. The plasma sample enters the reactor 10.

In step B, when the plasma sample reaches the first scale line 13 on the quantitative tube 4, the injection of the plasma sample into the quantitative tube 4 is stopped, and meanwhile, the quantitative plunger 5 moves down along the axis of the quantitative tube 4 and pushes the plasma sample down; and when the plasma sample in the quantitative tube 4 reaches the second scale line 14 below the first scale line 13, the plasma sample reaches the bottom of the quantitative tube 4.

The controller 9 calculates the stroke of the quantitative plunger 5 to push a certain amount of plasma sample out of the quantitative tube 4, and the controller 9 drives the quantitative plunger 5 by the driving mechanism 11 according to the calculated stroke.

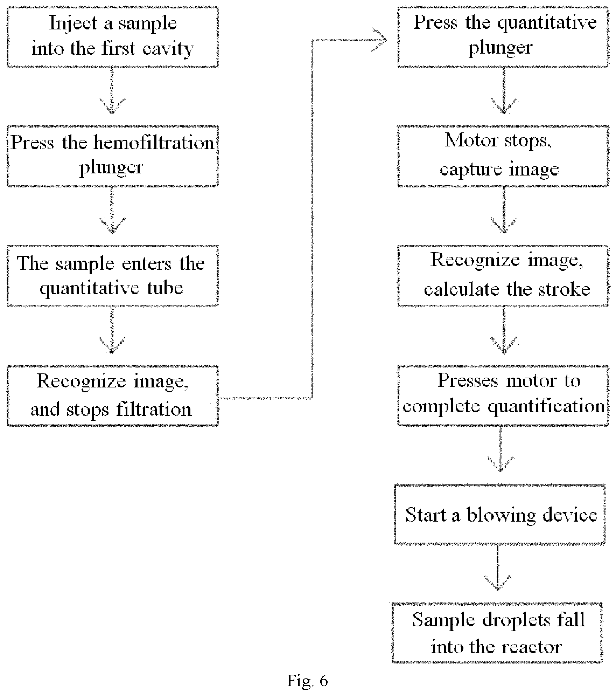

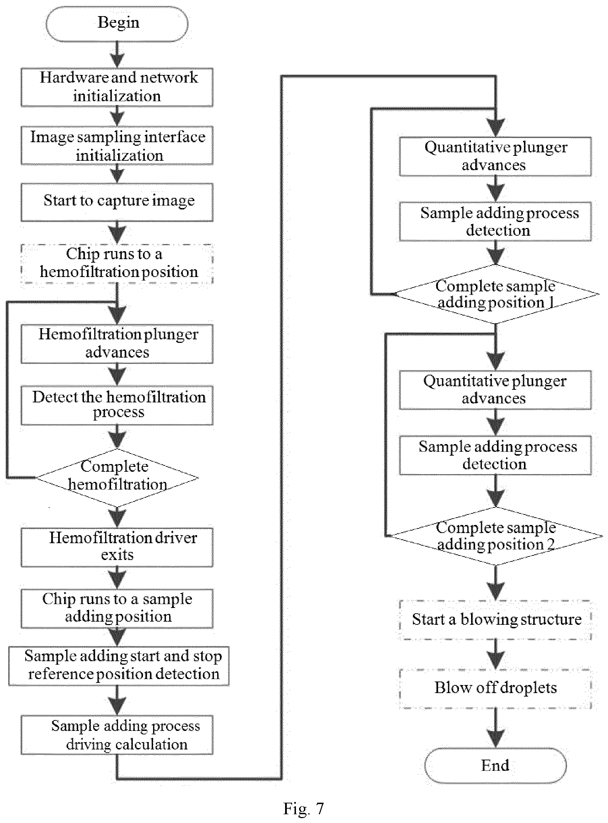

As shown in Figs. 6 and 7, a specific micro quantitative sampling method of the present invention is as follows:
Step 1, a whole blood sample is injected into the first cavity 101 via a feed port 15.
Step 2, when the biochip is detected, the controller 9 controls the driving mechanism 11 to drive the hemofiltration plunger 3 to move down.
Step 3, after the whole blood sample is filtered by the filtration membrane 2, the plasma sample enters the inner cavity of the quantitative tube 4 via the second cavity 102 and the sample injection port 401.
Step 4, the plasma sample reaches the first scale line 13 on the quantitative tube 4, the camera unit 8 captures a plasma sample image and transmits the image to the controller 9, the controller 9 recognizes the image and then controls the hemofiltration plunger 3 to stop pressing, and the filtration process ends.
Step 5, the controller 9 controls the driving mechanism 11 to drive the quantitative plunger 5 to move down to pre-press the plasma sample.
Step 6, the plasma sample reaches the second scale line 14 on the quantitative tube 4, the camera unit 8 captures a plasma sample image and transmits the image to the controller 9, the controller 9 recognizes the image and then controls the quantitative plunger 5 to stop pressing, and the filtration process ends.
Step 7, the controller 9 calculates a stroke of further pressing of the quantitative plunger 5 for taking a certain amount of plasma sample, and issues an instruction to instruct the driving mechanism 11 to drive the quantitative plunger 5 to move down according to the calculated stroke and push out the required sample amount. In this embodiment, the stroke for driving the quantitative plunger 5 is calculated based on the cross-sectional area of the inner cavity of the quantitative tube 4 at the sampling position.
Step 8, after the quantitative plasma sample is quantitatively discharged from the quantitative tube 4, micro quantitative (less than 50 microliters) sample droplets are adhered to the sample outlet 402 of the quantitative tube 4, the droplets are hung at the sample outlet 402, air blown from the air outlet of the air pump is controlled, and after the blown air passes through the blowing through hole 106, the air flowing down is gathered by the air passage 7 and blown to upper parts of the droplets, so that the droplets vertically fall down to the reactor 10, and the droplets will not be scattered and splashed out of the reactor 10 or blown to the side wall of the reactor 10, which ensures the quantitative precision of the sample. The output amount of the air pump is controlled by the controller 9 based on the volume of droplets calculated from the droplet image captured by the camera unit 8.

The embodiments of the present invention are described above with reference to the drawings, but the present invention is not limited to the specific embodiments. The specific embodiments described above are merely illustrative but not restrictive. Many forms may also be made by those of ordinary skill in the art under the enlightenment of the present invention without departing from the purpose of the present invention and the scope of the claims, and these forms fall into the scope of the present invention.

## Claims

1. A micro liquid sample loading structure, comprising a quantitative tube (4) and a hemofiltration structure, wherein a quantitative plunger (5) for sealing an inner cavity of the quantitative tube (4) is arranged at a top of the quantitative tube (4), a sample injection port (401) communicated with the inner cavity of the quantitative tube (4) is formed at an upper part of a side wall of the quantitative tube (4), and a sample outlet (402) is formed at a bottom of the quantitative tube (4); and a plasma outlet of the hemofiltration structure is communicated with the sample injection port (401);
the hemofiltration structure comprises a housing (1), a filtration membrane (2) is arranged in the housing (1), a top of the housing (1) is provided with a first cavity (101) with an upward opening, a second cavity (102) is formed in the housing (1), the first cavity (101) is communicated with the second cavity (102) by the filtration membrane (2), and the filtration membrane (2) is perpendicular to a horizontal plane; and the second cavity (102) is communicated with the sample injection port (401).

2. The micro liquid sample loading structure according to claim 1, wherein the bottom of the quantitative tube (4) is conical, and the sample outlet (402) is arranged at a lowest point of the bottom of the quantitative tube (4).

3. The micro liquid sample loading structure according to claim 1, wherein the quantitative tube (4) and the hemofiltration structure are integrated.

4. The micro liquid sample loading structure according to claim 1, wherein height of the sample injection port (401) is 1 mm to 2.5 mm, and the diameters of a middle section and an upper section of the quantitative tube (4) are 0.5 mm to 3.5 mm.

5. The micro liquid sample loading structure according to claim 1, wherein a hematocrit cavity (103) is further formed in the housing (1), the hematocrit cavity (103) is communicated with the bottom of the first cavity (101), the volume of the hematocrit cavity (103) is smaller than the volume of the first cavity (101), the hematocrit cavity (103) is communicated with the second cavity (102) by the filtration membrane (2), and the volume of the hematocrit cavity (103) is greater than or equal to the total volume of red blood cells in whole flood to be filtered.

6. The micro liquid sample loading structure according to claim 1, further comprising a hemofiltration plunger (3) that can seal the top opening of the first cavity (101).

7. The micro liquid sample loading structure according to claim 5, wherein a first highest point, on the side of the filtration membrane (2) where the filtration membrane (2) is connected to the hematocrit cavity (103), is on the same horizontal plane as a second highest point on the opposite side of the filtration membrane (2); or the first highest point, on the side of the filtration membrane (2) where the filtration membrane (2) is connected to the hematocrit cavity (103), is higher than the second highest point on the opposite side of the filtration membrane (2).

8. A biochip, comprising a reactor (10), wherein further comprising the micro liquid sample loading structure according to any one of claims 1 to 7, the sample outlet (402) is located above the reactor (10).

9. The biochip according to claim 8, wherein further comprising a chip substrate (12), the reactor (10) and the hemofiltration structure are arranged in the chip substrate (12); the quantitative tube (4) is arranged in the chip substrate (12); the sample outlet (402) is located above the reactor (10); the biochip further comprises a controller (9) and a driving mechanism (11) that can drive the quantitative plunger (5) to move along the axis of the quantitative tube (4), the controller (9) is electrically connected to the driving mechanism (11), and the driving mechanism (11) is connected to the quantitative plunger (5) in a driving manner.

10. The biochip according to claim 9, wherein further comprising a camera unit (8), the quantitative tube (4) is made of a transparent material and located within the field of view of the camera unit (8), and the camera unit (8) is electrically connected to the controller (9).

11. The biochip according to claim 10, wherein further comprising a blowing unit (6), the housing (1) is provided with a mounting through hole (105), the lower section of the quantitative tube (4) extends into the mounting through hole (105), a side wall of the housing (1) is provided with a blowing through hole (106) that communicates an air outlet of the blowing unit (6) with the lower section of the quantitative tube (4), and an air passage (7) that communicates the blowing through hole (106) with the outer wall of the sample outlet (402) is formed between the lower section of the quantitative tube (4) and the mounting through hole (105).

12. A micro quantitative sampling method for the biochip according to any one of claims 8 to 11, comprising the following steps:
step A. injecting a plasma sample into the inner cavity of the quantitative tube (4) from the sample injection port (401);
step B. the plasma sample reaching the bottom of the quantitative tube (4);
step C. the quantitative plunger (5) moving down along the axis of the quantitative tube (4) to push a certain amount of plasma sample out from the quantitative tube (4); and
step D. the plasma sample entering the reactor (10).

13. The micro quantitative sampling method for the biochip according to claim 12, wherein in step B, when the plasma sample reaches a first scale line (13) on the quantitative tube (4), the injection of the plasma sample into the quantitative tube (4) is stopped, and the quantitative plunger (5) moves down along the axis of the quantitative tube (4) and pushes the plasma sample down; and when the plasma sample in the quantitative tube (4) reaches a second scale line (14) on the quantitative tube (4) below the first scale line (13), the plasma sample reaches the bottom of the quantitative tube (4).

14. The micro quantitative sampling method for the biochip according to claim 12 or 13, wherein the controller (9) calculates the stroke of the quantitative plunger (5) to push a certain amount of plasma sample out of the quantitative tube (4), and the controller (9) drives the quantitative plunger (5) by the driving mechanism (11) according to the calculated stroke.

15. The micro quantitative sampling method for the biochip according to claim 12 or 13, wherein the plasma sample is obtained from whole blood by means of the hemofiltration structure.
